# FASCICULE DE BREVET EUROPEEN

(11) **EP 0 459 945 B1**
(45) Date de publication et mention de la délivrance du brevet: **19.11.1997**
(21) Numéro de dépôt: 91810378.9
(22) Date de dépôt: 17.05.1991
(51) Int. Cl.: A61N 1/36

(54) **Dispositif de stimulation électrique neuromusculaire**
Einrichtung zur neuromuskulären Elektroreizung
Neuromuscular electrical stimulation device

(30) Priorité: 26.05.1990 CH 1781/90; 20.12.1990 CH 4066/90
(43) Date de publication de la demande: 04.12.1991
(73) Titulaire: MED-EL Medical Electronics Elektro-medizinische Geräte GmbH, 6020 Innsbruck (AT)
(72) Inventeur: Brodard, Roland, CH-1844 Villeneuve (CH)
(74) Mandataire: Ganguillet, Cyril

(56) Documents cités:
- EP-A- 0 155 815
- EP-A- 0 197 889
- EP-A- 0 316 280
- US-A- 4 254 776

## Description

La présente invention concerne un dispositif de stimulation électrique neuromusculaire.

La stimulation électrique neuromusculaire consiste en une transmission transcutanée, par le biais d'électrodes de surface, de très faibles impulsions électriques au nerf et/ou au muscle visé pour en provoquer l'excitation. L'excitation obtenue est identique à celle qui est normalement provoquée par un influx nerveux physiologique autogène.

Selon l'intensité des impulsions et surtout leur schéma de distribution en fonction du temps, on peut définir deux options fondamentales: soit une action antalgique, soit une action motrice musculaire (contraction musculaire), dénommée électromyostimulation. L'électromyostimulation se divise à son tour en deux branches distinctes, celle du muscle sain, normalement innervé, et qui est alors une électromyostimulation fonctionnelle, et celle du muscle périphériquement dénervé, qui nécessite des paramètres de stimulation et un mode d'application totalement différents de la précédente.

Le but essentiel de l'électromyostimulation est de se substituer à une déficience momentanée ou durable de l'activité musculaire volontaire, de façon à en surmonter les conséquences, ou tout au moins à les atténuer, et à raccourcir autant que possible le délai de récupération de l'activité volontaire.

Le principe de la stimulation électrique neuromusculaire n'est pas récent. En 1791 déjà, le savant Luigi Galvani en a jeté les bases par ses expérimentations réussies de stimulation électrique du muscle de la grenouille. Or, depuis lors et pratiquement jusqu'à nos jours, l'électrostimulation n'a que peu progressé. On a essentiellement expérimenté différentes formes de courants de stimulation auxquelles des vertus thérapeutiques incertaines et décevantes ont été attribuées et qui n'étaient supportées que par des observations empiriques.

Cependant, au cour des dix dernières années, l'étude de la physiologie du muscle a connu un formidable essor. On a découvert que le muscle squelettique est un tissu extrêmement plastique, hétérogène et adaptable.

Les différents types de fibres qui constituent un muscle ont été arbitrairement et très schématiquement répartis en deux groupes fondamentaux:
- les fibres de type I, à contraction lente, peu puissantes, mais résistantes à la fatigue. Ces fibres ont une fonction posturale et sont également sollicitées lors d'une activité sportive d'endurance.
- les fibres de type II, à contraction rapide, puissantes mais fatigables. Ces fibres sont fortement impliquées dans les activités sportives de puissance de faible durée.

Ces deux types de fibres travaillent dans des plages de fréquences de contractions différentes et spécifiques. Ainsi, la plage de fréquences opérationnelles de la fibre I s'étend sur une plage de l'ordre de 8 à 25 Herz, tandis que celle de la fibre de type II s'étend sur une plage de l'ordre de 25 à 65 Herz.

Toutefois, compte tenu de la connaissance la plus récente de la physiologie musculaire, une telle classification des fibres se révèle beaucoup trop sommaire et arbitraire. En fait, les grandes différences de l'activité fonctionnelle musculaire développée durant la vie quotidienne suggèrent la nécessité d'une variabilité nettement plus étendue des profils métaboliques, dans différentes fibres musculaires. La réalité en a été maintenant démontrée par les méthodes ultramodernes d'analyses hystochimiques plus évoluées, qui ont permis d'affiner l'analyse et d'établir un dosage enzymatique fin et précis au sein de chaque fibre musculaire.

On a ainsi pu montrer que toute demande fonctionnelle donnée détermine une contraction musculaire caractérisée par quatre paramètres: sa force, sa vitesse, sa durée et sa fréquence de répétition.

La combinaison des différentes valeurs prises par ces paramètres sollicitera dès lors l'une des trois voies suivantes du métabolisme énergétique du muscle concerné: la voie aérobie, la voie anaérobie alactique ou la voie anaérobie lactique. Comme les profils morphologiques (structuraux) et métaboliques (biochimiques) d'une fibre musculaire, propres à assurer une voie aérobie ou au contraire anaérobie, divergent considérablement, il s'ensuit que non seulement il n'existe pas un type unique et universel de fibres au sein du muscle, mais on constate de plus que les fibres musculaires ne peuvent pas être classées, de façon rigide, dans les deux types décrits précédemment.

Au contraire, les fibres musculaires forment un spectre continu de fibres, avec différents niveaux d'organisation métabolique, ajustées à une large étendue de demandes fonctionnelles. De plus, le tissu musculaire se trouve de façon permanente en équilibre dynamique entre une dégradation (catabolisme) et une synthèse nouvelle (anabolisme). Ce même équilibre dynamique régit la morphologie et le profil métabolique qui caractérisent chacun des phénotypes des fibres musculaires composant un muscle donné. C'est encore ce même équilibre dynamique qui confère au muscle son extraordinaire plasticité: bien que la répartition initiale des différentes fibres musculaires au sein d'un muscle donné soit d'origine génétique, cette répartition n'est pas immuable, mais elle peut être modifiée, tant par une innervation croisée, que par une demande fonctionnelle spécifique constante imposée au muscle ou encore par une électromyostimulation chronique. Par conséquent, le phénotype d'une fibre musculaire est déterminé par un ensemble de réponses adéquates à des demandes fonctionnelles constantes. Certains schémas prédominants d'utilisation pour la locomotion ou la posture devraient favoriser l'expression de certains phénotypes prédominants.

Au vu des considérations qui précèdent, il n'est pas surprenant que le muscle contienne un spectre de fibres parmi lesquelles certaines espèces forment des populations limitées, constituant des entités métastables, correspondant aux types de fibres jusqu'alors généralement décrits.

On a ainsi pu montrer sur la base des connaissances récentes de la physiologie musculaire que l'efficacité, bien réelle, de l'électromyostimulation est une fonction directe du degré de mimétisme qu'elle respecte de l'activité volontaire équivalente à laquelle elle se substitue.

D'autre part, les séances traditionnelles d'électrothérapie, hebdomadaires ou bihebdomadaires, sont aujourd'hui totalement dépassées au profit de programmes de traitement quotidiens de plus longue durée. Une telle stimulation quotidienne implique une prise en charge du traitement par le patient lui-même en milieu hospitalier ou à la maison, sous contrôle médical périodique.

On connaît déjà des installations de stimulation électrique neuromusculaire comprenant un appareil programmeur et un appareil stimulateur fonctionnant indépendamment l'un de l'autre. L'appareil programmeur de ces installations est agencé pour enregistrer un programme de traitement sur un support d'information à mémoire morte, ce support d'information porteur du programme étant placé dans l'appareil stimulateur pour le commander et faire exécuter le programme. De telles installations sont décrites par exemple dans le brevet français no 1213080, dans la demande de brevet allemande publiée no 2903392, dans la demande de brevet européen publiée no 0087617 et dans la demande de brevet française publiée no 2528709.

L'inconvénient majeur de ces installations est qu'elles sont conçues avec des mémoires statiques, c'est-à-dire que les paramètres de stimulation programmés sont fixes et ne varient pas en fonction du temps de traitement. De ce fait, pendant toute la durée d'un traitement, le muscle est soumis à un régime métabolique uniforme et constant, aucun des appareils décrits dans ces publications ne permettant de tenir compte des conditions physiologiques rappelées plus haut et qu'il est nécessaire de respecter pour obtenir un entraînement musculaire efficace et exempt d'altération du tissu musculaire.

Une installation plus évoluée, comprenant un appareil programmeur (également agencé pour enregistrer un programme de traitement sur un support d'information amovible, mais à mémoire vive) pouvant travailler on-line avec un appareil stimulateur est décrit dans les demandes de brevet européens publiées nos 0197889 et 0316280. La technique exposée dans ces demandes de brevet est basée sur une hypothèse de travail aujourd'hui dépassée, qui se fonde sur la distinction mentionnée plus haut entre fibres musculaires dites lentes et fibres musculaires dites rapides. Cette installation est agencée pour une programmation séquentielle, la durée d'un traitement pouvant être divisée en un certain nombre de séquences distinctes, chacune pouvant être programmée, de façon à générer des trains d'impulsions électriques dont les caractéristiques sont adaptées aux fibres musculaires dites lentes, et d'autres trains d'impulsions électriques dont les caractéristiques sont adaptées aux fibres musculaires dites rapides.

L'inconvénient majeur de l'installation décrite dans ces deux demandes de brevet européen est que la valeur de chaque paramètre de stimulation programmé reste fixe à l'intérieur de chaque séquence et que cette valeur ne peut être que brutalement modifiée à l'occasion de la commutation d'une séquence à l'autre. De telles variations brutales et sans transition d'un régime spécifique de travail musculaire à un autre ne reflètent guère des schémas réels de l'activité musculaire volontaire de la vie quotidienne.

Cet inconvénient se traduit même par un risque majeur d'endommagement du tissu tendino-musculaire, quand on sait que l'intensité de la force délivrée par une contraction musculaire est la résultante des deux phénomènes physiologiques couplés suivants:
1) l'intensité de la force est fonction du nombre d'unités motrices qui sont recrutées et donc stimulées à un moment donné: c'est la "sommation spatiale", fonction de l'intensité du courant électrique de stimulation.
2) l'intensité de la force est également fonction de la fréquence des stimuli d'une unité motrice donnée: c'est la "sommation temporelle".

Or on sait qu'entre une simple suite de secousses musculaires non-fusionnées à basse fréquence subtétanique (par exemple à 8 Herz) et une fréquence déterminant une pleine fusion tétanique (par exemple à 25 Herz pour les fibres de type I et à 65 Herz pour les fibres de type II), la force délivrée pour une même intensité du courant électrique de stimulation augmente selon un facteur de 4 à 5!

C'est précisément pour cette raison que, dans l'installation précitée, lorsque la fréquence programmée augmente entre deux séquences consécutives, la commutation d'une séquence à l'autre ne peut pas être automatique: le déroulement du traitement est alors interrompu, tandis que le courant électrique est automatiquement coupé. L'utilisateur est alors contraint de redémarrer le traitement au début de la séquence suivante et de réajuster l'intensité du courant en fonction de la nouvelle fréquence programmée, de façon à déterminer une intensité tolérable de la force délivrée par le nouveau type de contraction musculaire. Cette manière de faire est d'autant plus fastidieuse que le programme est multiséquentiel.

On sait maintenant que, lors d'un développement volontaire de la force musculaire, il y a interaction et couplage entre la "sommation spatiale" et la "sommation temporelle" décrites ci-dessus. Très schématiquement, on peut dire, qu'en fonction de la force initiale à développer, un certain nombre d'unités motrices sont simultanément recrutées et se contractent à une fréquence donnée; puis au fur et à mesure que la force délivrée augmente, ladite fréquence s'élève et de nouvelles unités motrices additionnelles sont recrutées, tandis que la fréquence de contraction de l'ensemble des unités motrices recrutées continue d'augmenter. On remarque toutefois que dans ce mécanisme complexe d'interaction et de couplage des sommations spatiale et temporelle, la première reste prédominante en regard de l'intensité de la force délivrée par le muscle.

D'autre part, pour des raisons physiologiques, qui sortent du cadre de la présente invention, les différentes sorties vers les électrodes d'un stimulateur multicanaux de sortie devraient toujours être électriquement indépendantes, c'est-à-dire isolées galvaniquement entre elles et de la terre, et par conséquent dites flottantes. Cela requiert l'utilisation d'un dispositif d'isolation dans lequel la tension du stimulateur est découplée de la terre. Généralement ce dispositif est constitué par un transformateur. Cependant, compte tenu des propriétés électrophysiques d'un transformateur la durée de transmission d'une impulsion rectangulaire de courant constant ne peut guère être prolongée au-delà de 400 microsecondes, même au prix d'une meilleure qualité et d'une augmentation du volume dudit transformateur. Cette restriction limite considérablement les performances de l'électrostimulation quine peut dès lors pas être appliquée à des muscles périphériquement dénervés, dont la stimulation nécessite impérativement des impulsions d'une durée comprise entre 300 et 500 millisecondes.

Or, à ce jour, aucun des stimulateurs portables autonomes et multicanaux connus ne remplit les conditions énoncées ci-dessus, de façon à pouvoir stimuler aussi bien des muscles normalement innervés que des muscles périphériquement dénervés.

Le but de la présente invention est de proposer un dispositif de stimulation électrique neuromusculaire, sous la forme d'un appareil autonome portatif miniaturisé, qui soit exempt des défauts qui viennent d'être énumérés et qui permette de satisfaire les exigences que les dernières connaissances acquises en physiologie neuromusculaire ont révélées nécessaires pour que l'électromyostimulation permette d'assurer le plus proche mimétisme d'un entraînement musculaire actif volontaire, tout en conservant une application des plus simples, assurée par un automatisme poussé.

A cet effet, l'invention concerne un dispositif de stimulation électrique neuromusculaire, tel que défini à la revendication 1.

D'autres caractéristiques importantes de l'invention sont définies dans les revendications subordonnées à la revendication 1.

Les avantages de l'invention ressortiront bien de la description qui suit, donnée à titre d'exemple, et qui se réfère au dessin annexé.
- La fig. 1a: est une vue d'ensemble schématique, en perspective, d'une première forme d'exécution du dispositif, comprenant un élément stimulateur 2 et un ordinateur 1 reliés par un câble amovible 3.
- La fig. 1b: est une vue d'ensemble schématique, en perspective, d'un élément stimulateur 50 selon une seconde forme d'exécution du dispositif, et sa liaison avec d'éventuels éléments prériphériques.
- La fig. 2: est un schéma-bloc de l'élément stimulateur de la figure 1.
- La fig. 3: illustre à titre d'exemple deux formes distinctes de l'impulsion, montrant la variation de son amplitude i en fonction de sa durée a, a′.
- La fig. 4: illustre la fréquence de répétition des impulsions en fonction du temps t, exprimée en Herz, soit en nombre n d'impulsions par secondes s.
- La fig. 5: est un diagramme en fonction du temps t d'un train d'impulsions, comprenant un temps d'établissement et un temps d'extinction.
- La fig. 6: illustre deux trains d'impulsions consécutifs et la pause de durée h qui sépare ces deux trains.
- La fig. 7: est un diagramme qui illustre un exemple de paramètres interactifs en fonction du temps t de déroulement d'un programme de traitement.
- La fig. 8: est un diagramme illustrant un ensemble de trains d'impulsions e formant une série o.
- La fig. 9: illustre deux séries o₁ et o₂ consécutives et la période p caractérisant un intervalle entre deux séries consécutives.
- La fig. 10: est un diagramme qui illustre un exemple de commande externe et de paramètres interactifs entre deux canaux de sortie distincts, en fonction du temps t de déroulement du programme de traitement.

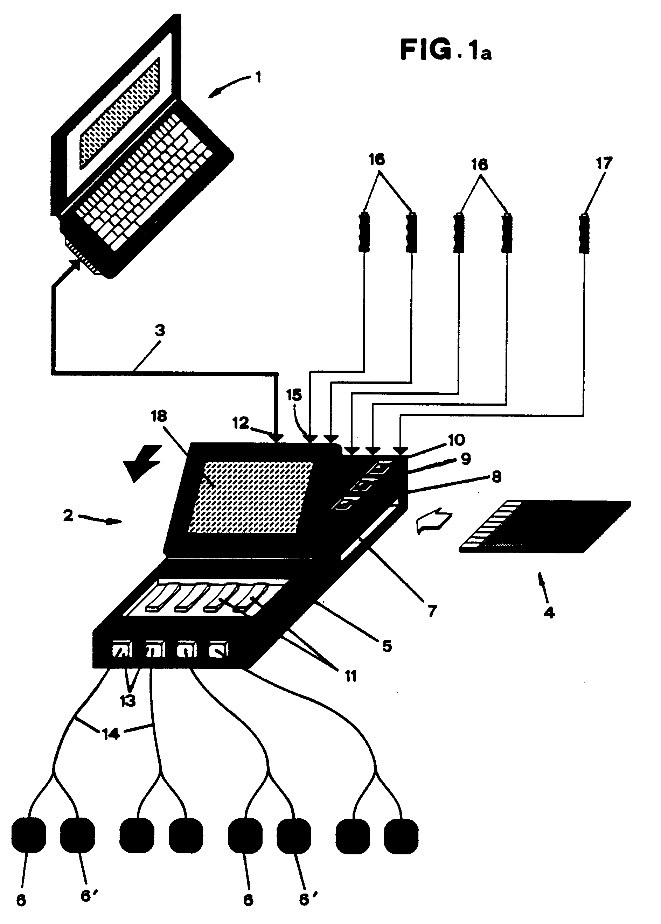

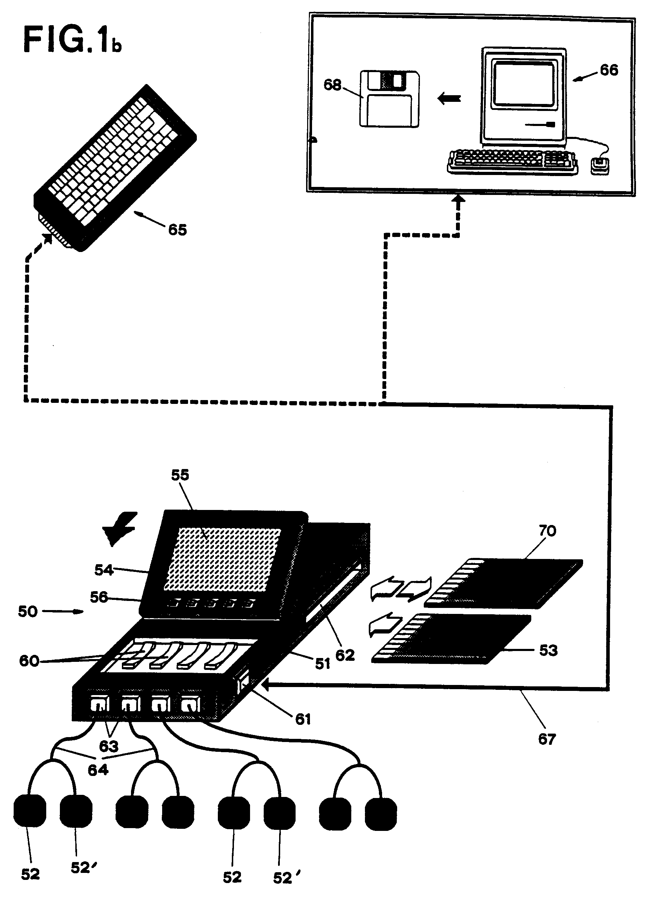

Le dispositif représenté à la figure 1a comprend un ordinateur du commerce 1 et un élément stimulateur 2 reliés par un câble amovible 3 assurant une liaison sérielle on-line RS 232C. L'élément de stimulation 2 est portatif et autonome. Il est commandé par un support d'information 4 amovible et interchangeable, préalablement programmable en fonction du traitement requis par chaque patient considéré. L'élément de stimulation est exécuté selon un concept du style baladeur miniaturisé. Son boîtier 5 contient les parties constitutives, mécaniques et électroniques. Il est muni de quatre paires d'électrodes 6, 6′ agencées pour être appliquées sur la partie à traiter du corps du patient et de façon à transmettre les trains d'impulsions électriques de stimulation.

L'extérieur du boîtier 5 est agencé de façon à mettre à disposition de l'utilisateur les éléments suivants:
- un écran 18 d'affichage LCD du type à matrice de points (dot matrix), rabattable de façon à constituer un couvercle de protection, fonctionnant en mode TV et géré tant par le logiciel d'exploitation que par le logiciel constituant le programme de traitement. Un tel dispositif, affichant aussi bien des caractères alphanumériques que des images graphiques, permet le contrôle de toutes les fonctions de l'appareil et de la stimulation, telles que paramètres de stimulation, durée du traitement, état de charge des batteries, barregraphes indiquant l'intensité du courant de stimulation de chaque sortie, forme d'impulsion utilisée, observance (compliance) du traitement par le patient, identification du programme de traitement chargé sur le support d'information connecté à l'appareil, instructions d'utilisation au patient, etc.,
- un interrupteur général 8 de mise en fonction de l'appareil,
- une commande 9 de démarrage et d'interruption momentanée du traitement,
- une commande 10 de suppression momentanée de la période de repos,
- des commandes 11 permettant d'augmenter ou de diminuer l'intensité du courant de stimulation de chaque sortie,
- un connecteur 12 permettant de raccorder le câble détachable multifonction, assurant la liaison avec le chargeur de batteries ou avec un organe de commande externe standard commune, ou encore la jonction sérielle on-line avec un ordinateur,
- un logement 7 d'insertion avec connecteur 23 pour le support d'information 4 amovible et interchangeable,
- des connecteurs 13 prévus pour le raccordement des câbles 14 de chaque paire d'électrodes 6, 6′, ainsi que des connecteurs 15 correspondant à ces derniers pour la commande externe 16 individuelle de chaque sortie.

Le dispositif de stimulation 2 est équipé d'un logement 7 et d'un connecteur pour recevoir la carte-mémoire, ainsi que d'un connecteur avec câble amovible 3 permettant une liaison sérielle du type RS 232C. Le dispositif de stimulation peut donc être relié sans autre à tout ordinateur muni d'une interface sérielle RS 232C. Il présente de ce fait l'avantage décisif de s'affranchir de la nécessité de tout appareil programmeur spécifique.

Pour charger un logiciel quelconque (d'exploitation ou de programme de traitement) sur une carte-mémoire, il suffit de connecter cette dernière au stimulateur en la plaçant dans son logement et de relier ledit stimulateur par sa liaison sérielle RS 232C à un ordinateur adéquat. Puis, par l'intermédiaire dudit ordinateur et de la liaison sérielle on-line ainsi établie, on peut à choix:
- transférer un logiciel d'exploitation de l'ordinateur sur la carte-mémoire et simultanément dans la mémoire interne du stimulateur,
- tranférer un logiciel de programme de traitement sur la carte-mémoire,
- effectuer la programmation libre d'un programme de traitement au moyen de l'ordinateur et simultanément charger ce programme sur la carte-mémoire,
- modifier un programme de traitement existant, préalablement transféré sur la carte de mémoire, et cela en fonction on-line, c'est-à-dire en temps réel pendant l'exécution même dudit traitement sur le patient.

Des cartes-mémoire, préalablement chargées d'un logiciel conformément à l'une des procédures décrites ci-dessus, peuvent bien entendu en tout temps être connectées à un stimulateur et exploitées par ce dernier de manière soit absolument autonome, soit en liaison sérielle on-line avec l'ordinateur.

On notera que la liaison sérielle autorise un véritable dialogue à double sens, dit écriture-lecture, entre l'ordinateur et le stimulateur. Dans le sens ordinateur-stimulateur, elle permet le transfert de logiciels et de paramètres et données prévus pour être ensuite traités par lesdits logiciels; tandis que dans le sens stimulateur-ordinateur, elle permet de relire des logiciels déjà transférés, ainsi que des paramètres et données mémorisés sur la carte-mémoire connectée au stimulateur. Un exemple en est fourni par l'observance, dont les données de rétrocontrôle délivrées par le stimulateur durant le déroulement du programme de traitement sont mémorisées sur la carte-mémoire.

Le stimulateur comprend un connecteur avec câble amovible assurant la liaison avec un organe pour la commande externe standard 17 commune à toutes les sorties et qui permet de contrôler d'une façon globale le début ou la fin d'une fonction donnée, définie par le logiciel. En outre, en plus de cette fonction générale, il comporte des connecteurs 15 avantageusement prévus pour la commande individuelle 16 de chaque canal de sortie. Cette commande individuelle permet, par le biais du logiciel spécifique chargé dans l'appareil, de commander le début ou la fin de la fonction du canal concerné et également de régler à distance l'intensité de la force de la contraction, en agissant sur l'intensité du courant et la fréquence des impulsions. Le couplage de cette commande à un capteur externe adéquat permet la transmission des informations au logiciel de l'appareil et d'assurer par exemple un entraînement fonctionnel correct de l'activité des membres du patients, sur la base des données fournies par ce rétrocontrôle (feedback and closed-loop control).

Selon une seconde forme d'exécution, représentée à la figure 1b, le dispositif comprend un élément stimulateur 50 portatif et autonome, conçu de façon à permettre une personnalisation simple de tout paramètre à l'aide de touches de fonction 56, permettant de s'affranchir de l'ordinateur 1 décrit plus haut. Comme l'élément stimulateur 5 de la figure 1a, cet élément est exécuté selon un concept du style baladeur miniaturisé. Son boîtier 51 contient les parties constitutives, mécaniques et électroniques. Il est muni de quatre paires d'électrodes 52, 52′ agencées pour être appliquées sur la partie à traiter du corps du patient et de façon à transmettre les trains d'impulsions électriques de stimulation. L'élément de stimulation est commandé par un support d'information 53 amovible et interchangeable, préalablement programmable en fonction du traitement requis par chaque patient considéré.

L'extérieur dudit boîtier 50 est agencé de façon à mettre à disposition de l'utilisateur les éléments suivants:
- un écran 55 d'affichage LCD du type à matrice de points (dot matrix), analogue à l'écran 18 du stimulateur de la figure 1a.
- des touches de fonction 56 (au nombre de cinq sur l'exemple représenté à la figure 1) à action fugitive, soumises au logiciel. Une fonction spécifique momentanée est assignée à chacune d'elles selon les besoins, cette fonction pouvant s'afficher sur une portion réservée du bas de l'écran,
- l'écran 55 et les touches de fonction 56 sont disposés sur un élément 54 rabattable de façon à constituer un couvercle de protection,
- des commandes à bascule 60 permettant d'augmenter ou de diminuer graduellement l'intensité du courant de stimulation de chaque sortie,
- un connecteur multifonctions 61 destiné à recevoir un câble détachable 67, permettant d'effectuer la liaison avec le chargeur de batteries ou une batterie d'alimentation d'appoint pour des traitements de très longue durée, ou avec un clavier 65, un ordinateur 66 ou tout autre dispositif de commande, ou encore la mise en réseau de plusieurs stimulateurs,
- un logement d'insertion 62 avec connecteur pour le support d'information 53 amovible et interchangeable,
- des connecteurs 63 prévus pour le raccordement des câbles 64 de chaque paire d'électrodes 52, 52′.

Le stimulateur 50 est agencé de façon que sa mise en fonction s'effectue à l'aide de l'une des touches de fonction 56. On peut par exemple assigner la fonction d'enclenchement à l'une quelconque des touches de fonction, cette touche redevenant disponible pour une autre fonction après l'enclenchement. On pourrait également prévoir que la mise en fonction du stimuateur s'effectue lorsqu'on appuie sur n'importe quelle touche de fonction, mais bien entendu cette mise en fonction peut également s'effectuer de toute autre manière adéquate. On peut d'autre part prévoir que la commande de démarrage et d'interruption momentanée du traitement et la commande de suppression momentanée de la période de repos s'effectuent à l'aide d'une touche de fonction dont le numéro est indiqué sur l'écran, par exemple sur sa partie inférieure.

Qu'il soit réalisé selon la forme d'exécution représentée à la figure 1a ou selon la forme d'exécution représentée à la figure 1b, le présent dispositif permet de rendre l'activité des différents canaux de sortie, programmables indépendamment les uns des autres, interdépendante au gré des besoins, tout paramètre d'un canal de sortie pouvant asservir un autre canal de sortie. Ceci autorise la programmation d'activités musculaires multiples, synergistes, alternées synergistes-antagonistes ou relayées complexes.

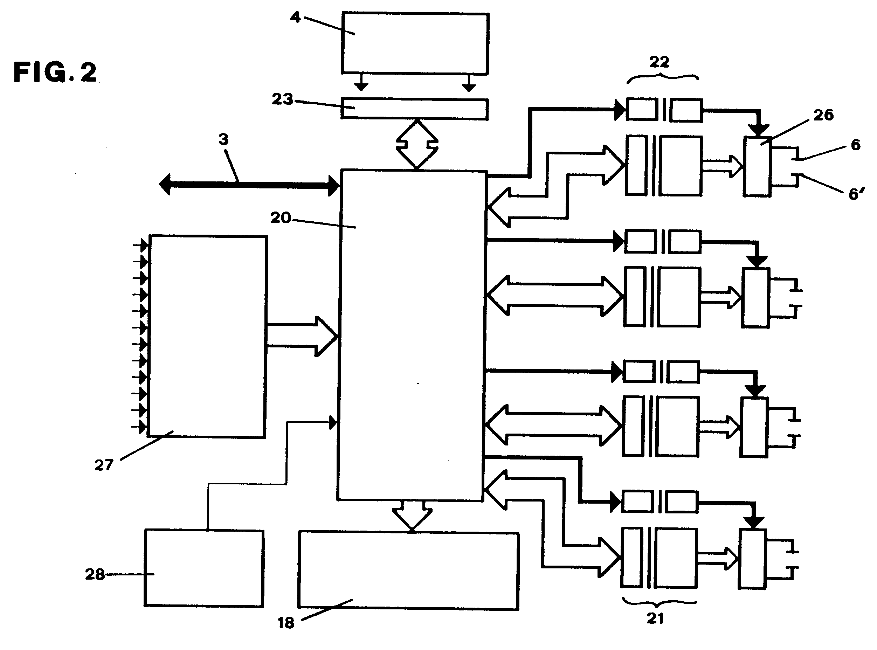

Comme représenté sur le schéma-bloc de la figure 2, qu'il s'agisse de l'une ou l'autre des formes d'exécution décrites ci-dessus, la circuiterie électronique (hardware) consiste essentiellement en un véritable micro-ordinateur 20 générateur d'impulsions électriques. Ce micro-ordinateur est constitué des éléments traditionnels: processeur, mémoires ROM (read only memory) et mémoires vives RAM (random access memory). Ce micro-ordinateur est servi par une interface 27 d'entrée des commandes manuelles et des commandes externes.

A partir de la forme originale de l'impulsion programmée (par exemple une impulsion rectangulaire de courant constant ou une impulsion de courant progressif triangulaire), le micro-ordinateur calcule et délivre des impulsions de haute fréquence à une véritable cellule d'isolation 21 comprenant un transmetteur inductif et un démodulateur, ce dernier comprenant un redresseur double alternance et une cellule de filtrage et lissage, de façon à constituer un amplificateur conformateur de sortie du courant de stimulation vers les électrodes; c'est-à-dire que le signal de haute fréquence ainsi obtenu est ensuite transmis et amplifié (survolté) inductivement, de façon indépendante pour chaque sortie; puis ce signal est redressé, filtré et lissé, de telle façon que la forme originale programmée de l'impulsion à la sortie de ce circuit soit de nouveau restituée.

Cependant, comme une telle impulsion redressée est forcément délivrée avec une polarité constante, l'étage redresseur a été complété par un étage commutateur-inverseur électronique 26 de la polarité, commandé par le logiciel, via une cellule d'isolation électrique 22, constituée par un transformateur ou un opto-coupleur.

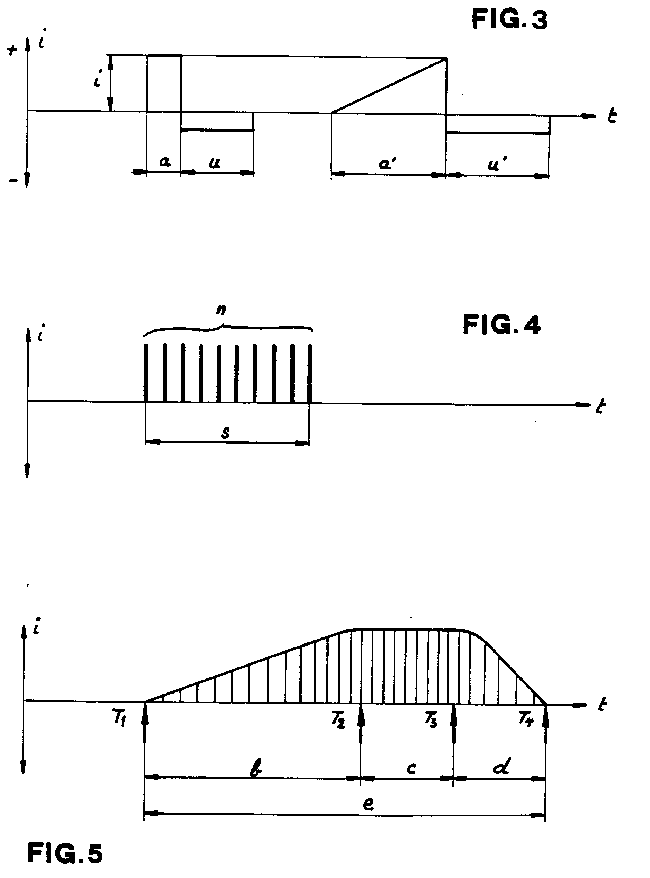

Cette méthode présente l'avantage de permettre la délivrance d'impulsions de courant constant ou à pente variable, telles que représentées sur la figure 3, programmées pour n'importe quelle durée, voire même pour fournir un véritable courant continu ininterrompu, destiné à l'ionothérapie, tout en gardant un contrôle permanent de la polarité du courant de sortie, ce qui autorise en outre et en particulier de générer des impulsions asymétriques, également dénommées compensées, parce que le flux de courant de l'impulsion active de stimulation a, a′ est alors totalement compensé par un flux de courant inverse u, u′ d'intensité inférieure inactive mais prolongée, avec pour avantage majeur un équilibrage (flux moyen nul) des charges électriques au niveau de la peau, ce qui est impératif pour prévenir toute brûlure ou altération électrolytique de cette dernière.

Cette conception générale présente l'avantage que l'ensemble de la circuiterie électronique (hardware) est du type amorphe, c'est-à-dire entièrement sous le contrôle du logiciel (software), ce qui assure une flexibilité maximale d'emploi et d'adaptation du système.

Le système fonctionne au moyen de deux logiciels distincts, soit un logiciel d'exploitation et un logiciel de traitement, ce dernier constituant le programme de traitement proprement dit, chargé et mémorisé sur le support d'information amovible et interchangeable 4, respectivement 53, chaque programme de traitement constituant ainsi un programme d'application différent. Le logiciel d'exploitation peut être chargé dans une mémoire vive interne de l'appareil, du type RAM, où il reste mémorisé en permanence, jusqu'à son éventuel remplacement lors du chargement d'un autre logiciel d'exploitation. Toutefois, selon un mode d'exécution préférentiel, le stimulateur ne comporte aucune mémoire résidentielle, le logiciel d'exploitation étant également mémorisé sur le support d'information amovible et interchangeable, d'où il dialogue en permanence avec le microprocesseur.

La circuiterie électronique est alimentée par des piles ou des batteries 28 rechargeables, incorporées dans le boîtier 5, respectivement 51.

La commande de l'intensité du courant de stimulation de chaque sortie est généralement effectuée au moyen d'un potentiomètre ou de deux touches à action momentanée, l'une (+) étant destinée à augmenter cette intensité, tandis que l'autre (-) diminue ladite intensité.

Avec le présent dispositif, l'intensité du courant, assignée initialement par le thérapeute, étant mémorisée par des moyens asservis au programme et au processeur, la commande manuelle 11, respectivement 60, de l'intensité est en fait une commande complémentaire, utilisée essentiellement pour corriger éventuellement l'intensité programmée. Une telle commande agit par conséquent également par des moyens asservis au programme et au processeur.

L'organe de commande du contrôle de l'intensité de chaque sortie est un commutateur unique 11, respectivement 60, à bascule à action momentanée. En position de repos, il est à l'état neutre inactif. L'avantage de ce dispositif est de ne nécessiter pour son actionnement que l'usage d'un seul doigt et de permettre ainsi un réglage précis et rapide de ladite intensité par un simple léger déplacement de ce doigt sur la touche.

Il est évident que le support d'information amovible et interchangeable peut être réalisé de différentes manières. Dans l'exemple décrit ici, il s'agit d'un élément dit carte à puce 4, respectivement 53, au format d'une carte de crédit, contenant des mémoires vives du type EEPROM (electrical erasable programmable read only memory) ou RAM (random access memory) et muni d'un connecteur. Cette carte-mémoire fonctionne comme une disquette d'ordinateur.

Le dispositif est agencé pour une programmation dynamique déroulante libre à paramètres interactifs. Le micro-ordinateur du stimulateur comporte un système d'horloges internes de référence, auquel sont asservis les logiciels utilisés. De par cette conception, l'utilisateur dispose d'une pleine capacité de programmation libre de tous les paramètres de stimulation en fonction du temps de déroulement du programme de traitement; cette programmation fonction du temps vaut aussi bien pour l'introduction et la mise en oeuvre d'un paramètre donné puis son éviction, que pour la valeur qui lui est assignée.

Pour faciliter la programmation et pour assurer une progressivité convenable de la variation de la valeur assignée à un paramètre donné en fonction du temps, il est prévu que l'utilisateur puisse fixer librement, par échantillonnage en fonction du temps, la valeur assignée à ce paramètre, soit arbitrairement en se référant à ses connaissances et son expérience, soit expérimentalement en utilisant la liaison sérielle on-line avec l'ordinateur ou les touches de fonction et en initialisant la valeur dudit paramètre en temps réel pendant le déroulement effectif du programme de traitement sur le patient, et de ce fait en fonction des réactions dudit patient.

La programmation du stimulateur étant une programmation déroulante libre à paramètres interactifs, il en résulte qu'en fonction du déroulement d'un programme de traitement, la valeur de tout paramètre de stimulation peut évoluer de manière continue et progressive entre des valeurs de référence préalablement assignées, tandis que tout paramètre donné peut en asservir un autre.

La fréquence des impulsions peut ainsi par exemple passer de façon continue et progressive d'un régime subtétanique à un régime pleinement tétanique, pendant que l'intensité du courant de sortie correspondant, asservie à la fréquence, est par conséquent corrigée et adaptée, de façon également continue et progressive, pour maintenir la force musculaire délivrée dans des limites quasiment constantes ou préalablement définies.

Une telle programmation permet de réaliser des programmes d'exercice musculaire qui respectent une progression continue et graduelle de l'intensité de l'activité musculaire développée, calquée sur la physiologie d'un entraînement volontaire similaire. Cette condition prend toute sa valeur lors de l'accomplissement d'une série de contractions, telles que par exemple lors d'un entraînement par intervalles.

Le connecteur 61 du stimulateur de la figure 1b peut être utilisé pour réaliser une liaison du type bus de données permettant la télécommande externe interactive des canaux de sortie d'un ou de plusieurs stimulateurs couplés en réseau. Dans ce dernier cas, la liaison peut être réalisée par une ligne sérielle multiple en étoile, avec un point central, le point central pouvant être constitué par un ordinateur ou tout autre dispositif de commande, ou par l'un des stimulateurs dans lequel on a introduit une carte-patient maître à laquelle sont asservies les cartes-patients des autres stimulateurs.

Dans le cas du stimulateur représenté à la figure 1b, la programmation du stimulateur peut être réalisée à l'aide de cartes-programmes 70 préétablies, serveuses de programmes standards de traitements, chaque programme étant spécifique à une indication médicale donnée précise. Lorsque cette carte a été introduite dans le logement d'insertion 62, une liste des programmes apparaît sur l'écran et un programme standard peut alors être choisi, soit à l'aide des touches de fonctions 56, soit à l'aide d'un clavier 65, d'un ordinateur 66 ou de tout autre dispositif de commande qui peut être connecté au stimulateur par l'intermédiaire de la ligne 67. Le programme choisi peut alors être copié sur une carte-programme-patient vierge que l'on introduit dans le logement d'insertion 62 après avoir retiré la carte-programmes 70. Le programme choisi peut ensuite être facilement personnalisé et adapté au cas particulier du patient, à l'aide des touches de fonction 56 ou de tout autre dispositif de commande connecté au stimulateur par la ligne 67, pendant l'exécution du traitement, en fonction des réactions du patient aux paramètres de stimulation proposés par le programme initial.

La présence, sur le stimulateur, des touches de fonction 56 asservies au logiciel, permet à l'utilisateur de jongler entre les cartes-serveuses et les cartes patients et de personnaliser le programme choisi à volonté, sans nécessiter une connexion à une unité de commande extérieure. Les avantages d'un tel agencement sont évidents, puisqu'ils confèrent à l'appareil une grande souplesse d'adaptation, qui se traduit par un grand confort d'utilisation.

Il est bien entendu également possible de personnaliser les programmes à l'aide de tout ordinateur personnel doté d'une liaison sérielle RS-232-C standard, et que l'on connecte au stimulateur par l'intermédiaire du câble 67, et même d'effectuer une programmation totalement libre, en utilisant des logiciels préparés à cet effet et livrés sur des disquettes 68.

Le logiciel utilise des fonctions d'interpolation adéquates, telles que par exemple des fonctions splines (fonctions à polynômes de degré variable de Tchebychev, Lagrange, Spline, etc.) qui permettent le calcul d'une courbe de variation de la valeur du paramètre entre les divers échantillonages successifs, de telle façon que cette valeur suive une courbe harmonieusement progressive et lissée. Ainsi, le dispositif présente l'avantage de permettre une variation progressive contrôlée et physiologique du type de travail imposé au muscle et également d'éviter une surcharge brutale et dommageable de l'appareil tendino-musculaire.

Un exemple de diagramme d'un train d'impulsions d'une durée e comprenant un temps d'établissement b et un temps d'extinction d est représenté sur la figure 5. La durée c d'intensité nominale des impulsions est égale à e-b-d. Ce diagramme illustre également la variation progressive programmable de la fréquence des impulsions, en fonction du temps t de déroulement du programme de traitement, entre les points T₁ et T₂ (pente d'établissement), puis T₃ et T₄ (pente d'extinction) d'échantillonnage-initialisation; la valeur de la fréquence et la courbe enveloppe lissée de l'intensité des impulsions entre ces différents points T étant calculées par des fonctions d'interpolation inclues au logiciel.

La conception totalement informatisée du stimulateur avec une circuiterie (hardware) de type amorphe, entièrement soumise à la commande et au contrôle du logiciel (software), permet par l'intermédiaire de ce dernier de prévoir des couplages interactifs entre les différents paramètres de stimulation en fonction du temps de déroulement d'un programme de traitement. Un exemple en est fourni ici par l'asservissement de l'intensité du courant de stimulation délivré à chaque sortie à la fréquence des impulsions électriques délivrées à cette même sortie.

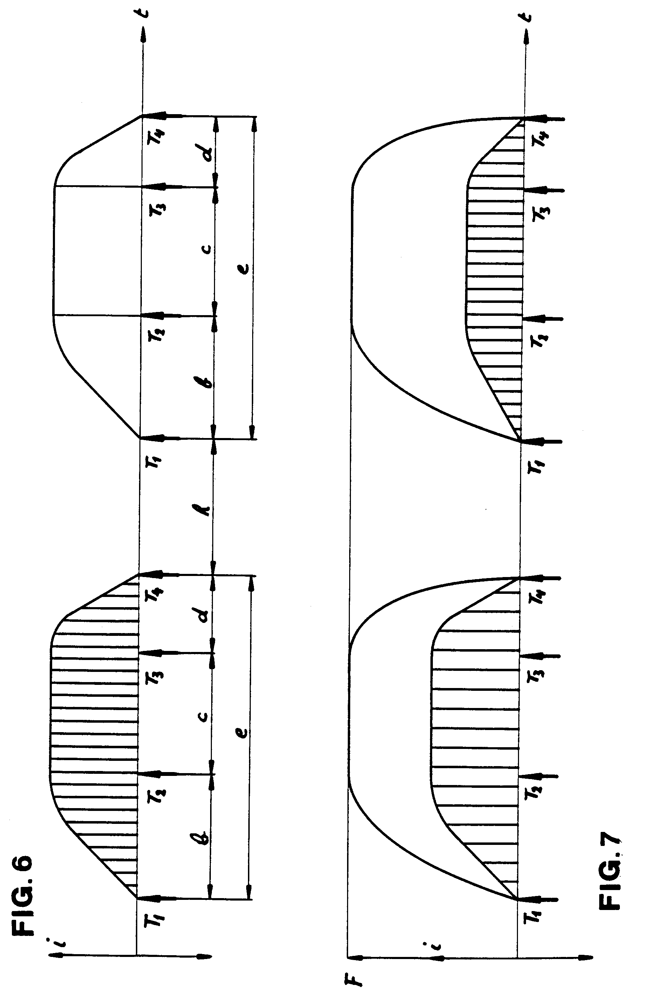

La figure 7 représente à titre d'exemple l'asservissement par le logiciel de l'intensité i des impulsions en fonction de la fréquence variable programmée des impulsions; la résultante, la force F délivrée par le muscle stimulé est ainsi maintenue constante.

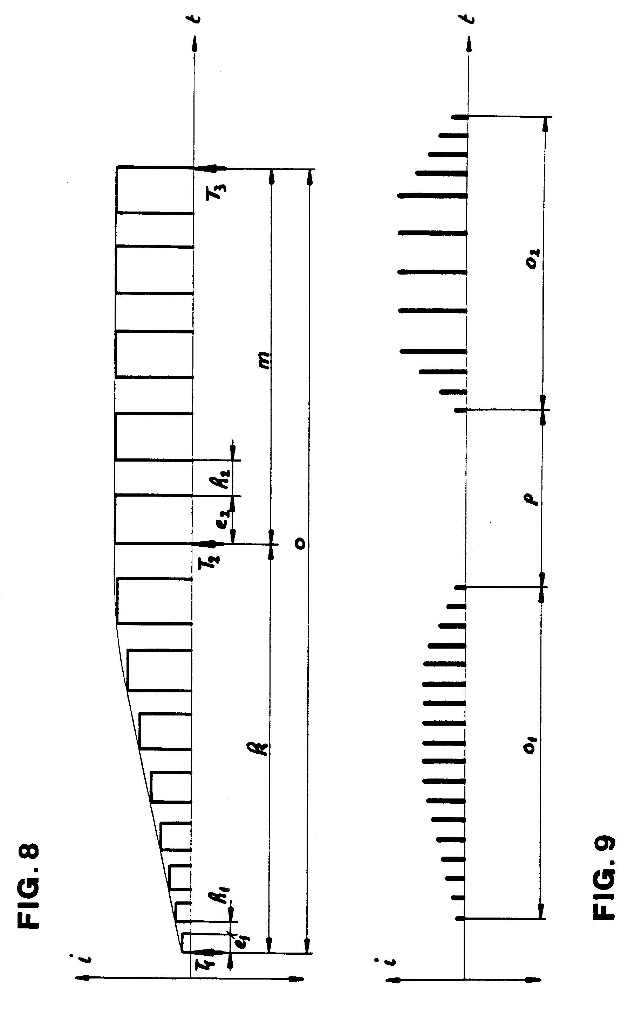

Un ensemble de trains d'impulsions e formant une série o est représenté sur la figure 8. Ce diagramme illustre également un exemple de paramètres interactifs, soit la variation progressive programmable, en fonction du temps t de déroulement du programme de traitement, de l'intensité du courant de stimulation i, de la durée des trains d'impulsions (de e₁ à e₂) et de la durée de la pause (de h₁ à h₂) entre les trains d'impulsions, et cela entre les points T₁, T₂ et T₃ d'échantillonage-initialisation; les valeurs respectives de ces paramètres et la courbe enveloppe lissée du courant de stimulation i étant calculées par des fonctions de couplage interparamètres et d'interpolation inclues au logiciel. Ce diagramme illustre de plus l'ensemble de trains d'impulsions contenus dans la rampe k de la série o, ainsi que la phase m de travail nominal.

Comme on l'a dit plus haut, la force délivrée par un muscle est la résultante de la combinaison de la sommation spatiale et de la sommation temporelle, c'est-à-dire, sur le plan de l'électrostimulation, de la combinaison de l'intensité de l'impulsion électrique et de sa fréquence de répétition. On peut même préciser que pour un muscle stimulé donné, la force délivrée est égale au produit de l'intensité du courant par la fréquence des impulsions, multiplié par un facteur variable expérimental de correction. Ce facteur variable, dont la valeur est une fonction de la fréquence des impulsions et de la structure des fibres composant le muscle considéré, peut être déterminé au moyen d'une table de corrélation établie de manière expérimentale et précisé pour chaque muscle par une simple procédure d'initialisation.

Il faut en effet rappeler ici que les fibres de type I montrent une tétanisation complète aux environs de 25 Herz, tandis que cette même tétanisation complète n'est atteinte qu'aux environs de 50 à 65 Herz pour les fibres de type II. Comme les différents muscles squelettiques contiennent des proportions variables de ces différents types de fibres musculaires et que de plus cette proportion varie encore d'un individu à l'autre, il est évident qu'il n'est guère possible d'établir une table de corrélation unique garantissant une approximation générale acceptable du facteur variable indiqué ci-dessus. Cependant cet inconvénient peut être facilement surmonté par une très simple procédure d'initialisation effectuée préalablement au traitement: le stimulateur en fonction sur le patient considéré est chargé avec le programme de traitement. A l'aide de l'ordinateur de programmation relié par sa liaison sérielle on-line ou à l'aide des touches de fonction, le thérapeute applique alors successivement à chaque sortie (paire d'électrodes) la procédure suivante: il délivre une fréquence d'impulsions subtétaniques de l'ordre de 8-10 Herz et élève l'intensité du courant de stimulation jusqu'au seuil maximal de tolérance du sujet; ces données sont alors mémorisées par le logiciel, puis l'intensité du courant étant ramenée à zéro, la fréquence est augmentée à 50-65 Herz et le thérapeute élève à nouveau l'intensité du courant jusqu'au seuil maximal de tolérance du sujet, ces données étant à leur tour mémorisées jar le logiciel. Ce dernier peut dès lors calculer, par une fonction d'interpolation adéquate et avec l'aide de la table de corrélation précitée, l'asservissement de l'intensité du courant de stimulation par la fréquence des impulsions, de telle façon que la force délivrée par le muscle stimulé reste constante lorsque la fréquence programmée des impulsions varie en fonction du déroulement du programme de traitement.

Ce qui vient d'être décrit ci-dessus implique évidemment que le réglage de l'intensité du courant de stimulation en fonction du temps de déroulement du programme de traitement est sous le contrôle du logiciel de programmation.

De plus, l'intensité du courant de stimulation délivré à chaque sortie est automatiquement et systématiquement enregistrée et mémorisée par le logiciel en fonction du temps de déroulement du programme de traitement. L'intensité ainsi mémorisée sera automatiquement restituée à l'occasion d'une application ultérieure dudit programme de traitement, et cela au fur et à mesure de son déroulement.

Bien que, comme décrit plus haut, l'intensité soit asservie à la fréquence des impulsions programmée, le patient conserve néanmoins en tout temps la faculté prédominante de modifier cette intensité, de façon purement manuelle, au moyen de la touche de commande prévue à cet effet. La nouvelle intensité ainsi assignée par le patient est à son tour enregistrée et mémorisée et les fonctions d'interpolation décrites précédemment recalculent alors automatiquement la nouvelle courbe des valeurs de ce paramètre en fonction du temps de déroulement du programme de traitement.

Dans l'exemple décrit ici le stimulateur comporte quatre sorties électriquement indépendantes, isolées galvaniquement entre elles et de la terre, et par conséquent dites flottantes. La programmation et le fonctionnement de chaque sortie, en fonction du temps de déroulement d'un programme de traitement, sont totalement indépendants. Ils peuvent toutefois être couplés de manière interactive par les couplages prévus par le logiciel entre les différents paramètres de stimulation en fonction du temps de déroulement du programme de traitement, comme décrit plus haut. En effet, ce couplage interactif des paramètres est également possible entre les paramètres des différents canaux de sortie.

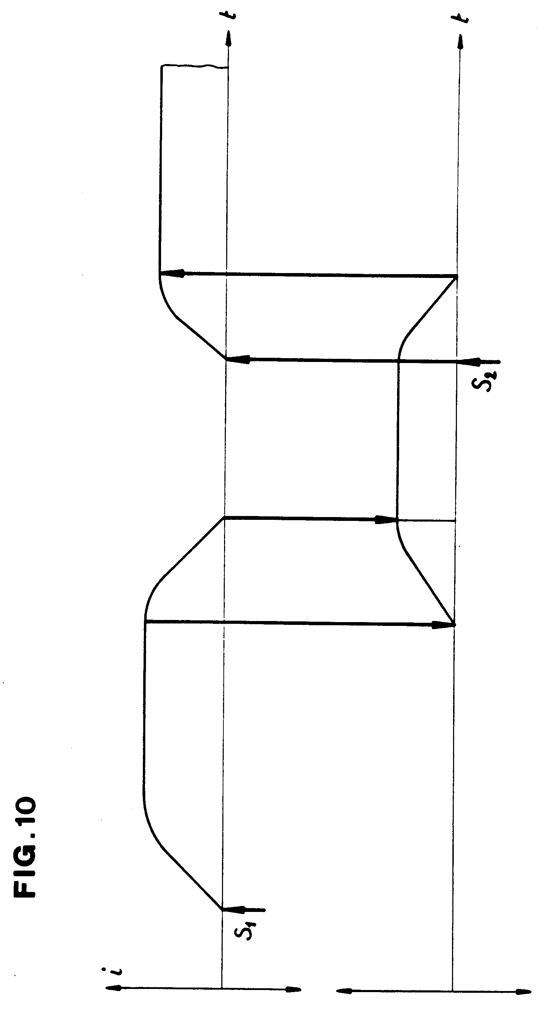

Un exemple de commande externe et de paramètres interactifs entre deux canaux de sortie distincts, en fonction du temps t de déroulement du programme de traitement est représenté sur le diagramme de la figure 10. La commande externe individuelle S₁ du premier canal détermine le début d'un premier train d'impulsions; la fin de celui-ci commande l'établissement d'un train d'impulsions du second canal; ce train d'impulsions est interrompu par la commande externe individuelle S₂ de ce second canal; la fin de ce dernier train d'impulsions commande alors l'établissement d'un nouveau train d'impulsions du premier canal.

Les principaux paramètres de stimulation sont les suivants:
- la forme de l'impulsion électrique, soit la variation de son amplitude en fonction de sa durée (figure 3),
- la durée a, a′ de l'impulsion,
- l'amplitude i de l'impulsion,
- la fréquence de répétition des impulsions en fonction du temps (figure 4),
- la durée e d'un train d'impulsions (figure 5),
- la durée h de la pause qui sépare deux trains d'impulsions consécutifs (figure 6),
- le temps b ou la pente d'établissement d'un train d'impulsions (figure 5),
- le temps d ou la pente d'extinction d'un train d'impulsions (figure 5),
- la durée ou le nombre caractérisant un ensemble de trains d'impulsions formant une série o (figure 8),
- la période p caractérisant un intervalle entre deux séries consécutives (figure 9),
- la durèe ou le nombre caractérisant un ensemble de trains d'impulsions contenus dans la rampe k d'une série (figure 8),
- la durée du programme de traitement.

Cette liste ne saurait être exhaustive, tant il est vrai que d'autres paramètres peuvent être constamment incorporés au logiciel à volonté, au gré des besoins.

On sait que les paramètres optimaux de stimulation et de contrôle du déroulement d'un programme de traitement varient grandement en fonction des schémas d'activité particuliers que le thérapeute veut imposer aux muscles, en accord avec ses objectifs thérapeutiques, spécifiques à chaque cas et à chaque patient, et que ces paramètres évoluent de plus constamment en fonction des progrès continus de la connaissance de la physiologie moderne du muscle.

La conception du dispositif de la présente invention, conçu avec une circuiterie (hardware) amorphe, entièrement sous la commande de logiciels (software), élaborés pour permettre une programmation dynamique déroulante libre à paramètres interactifs, permet précisément de programmer, à volonté et de façon pleinement évolutive, n'importe quels schémas interactifs envisageables d'activités musculaires, en mettant en jeu de plus à choix un métabolisme aérobie, anaérobie alactique ou anaérobie lactique, si complexes soient-ils, tout en garantissant au patient une application des plus simples, en raison de l'automatisme intégral du déroulement du programme de traitement, des plus sûres, en raison des dispositifs de sécurité décrits (fonctions d'interpolation et d'initialisation) et des plus fiables, en raison d'une observance absolue et incontournable.

Tel que décrit ci-dessus, le dispositif de l'invention permet aussi bien d'effectuer tous les traitements par électromyostimulation des muscles périphériques normalement innervés et des muscles périphériquement dénervés, que les traitements de stimulation nerveuse à but antalgique, ou encore les traitements d'ionothérapie (ionisation médicamenteuse), tels que par exemple:
- stimulation du muscle sain, normalement innervé;
- stimulation du muscle périphériquement dénervé;
- électrodiagnostic neuro-musculaire, établissement de la courbe intensité-durée, rhéobase, chronaxie, climalyse, dissociation des seuils par par la méthode de Fischgold, etc.;
- stimulation antalgique avec wobulation antiaccoutumance du signal;
- ionothérapie (ionisation médicamenteuse par courant continu contrôlé);
- entraînement de toute activité fonctionnelle, mêmecomplexe, grâce aux quatre canaux de sortie, programmables indépendamment les uns des autres, et à la conception modulaire du dispositif, avec "bus de données" qui permet la télécommande interactive des canaux de sortie d'un ou de plusieurs stimulateurs couplés en réseau;
- etc..

L'observance ou compliance du traitement consiste en un rétrocontrôle de la bonne exécution du programme de traitement par le patient. Ce rétrocontrôle de certains paramètres significatifs dudit traitement, tels que sa durée, son intensité, etc., est défini par le logiciel et il est mémorisé sur le support d'information amovible et interchangeable connecté au stimulateur. Cette observance constante peut ainsi en tout temps être relue et interprétée par le thérapeute. Une observance fiable est un élément essentiel du traitement prescrit, tant pour garantir la crédibilité des études cliniques que pour rassurer les tiers payeurs que sont généralement les assurances, quant au bon emploi des appareils par les patients à la maison.

On connaît déjà quelques stimulateurs munis de quelque dispositif d'observance. Cependant, les dispositifs proposés sont par trop facilement détournables par le patient, puisqu'il lui suffit d'effectuer une simple mise en court-circuit de chaque paire d'électrodes (donc sans les placer sur son corps) pour tromper l'observance et donner ainsi l'illusion au thérapeute d'avoir parfaitement exécuté son programme de traitement.

La résolution de ce problème est complexe car l'impédance en court-circuit des électrodes est en fait très proche de celle révélée sous charge corporelle pendant une impulsion électrique de stimulation. En effet, pour des raisons physiologiques, la durée moyenne d'une telle impulsion est de l'ordre de seulement 300 microsecondes pour la stimulation des muscles normalement innervés. L'utilisation d'une durée aussi courte permet entre autres au courant un passage transcutané essentiellement par effet capacitif plutôt qu'ohmique, en ne lui opposant ainsi qu'une faible impédance.

Le dispositif de la présente invention garantit une solution fiable à ce problème. L'impédance de la charge entre une paire d'électrode est constamment mesurée pendant toute la durée du traitement. Si sa valeur dépasse une valeur limite, assignée par le logiciel, le courant de stimulation s'interrompt automatiquement et un symbole spécifique est affiché sur l'écran de contrôle LCD du stimulateur. Ce dispositif permet de détecter et d'éliminer les effets néfastes de tout mauvais contact, tant au niveau des connecteurs, des câbles, des électrodes, que de la peau elle-même. Par conséquent, s'il n'y a pas de charge entre les électrodes, il est évident que le traitement ne peut pas être démarré.

De plus, lorsque l'utilisateur actionne la touche de démarrage d'un programme de traitement, préalablement au début de la stimulation propement dite, le dispositif génère entre les électrodes une impulsion de courant constant d'une durée de quelques millisecondes, mais avec une très faible intensité de courant, largement subliminaire par rapport au seuil de sensibilité du patient. Comme le courant constant de cette impulsion charge la capacité cutanée au niveau des électrodes, il s'ensuit que la tension nécessaire pour fournir cette impulsion s'élève progressivement selon une certaine pente. On rappellera pour mémoire que si la charge entre les électrodes est une résistance purement ohmique, la tension reste alors constante. Il suffit dès lors de comparer la valeur de la pente de la tension mesurée à une valeur de référence introduite dans le logiciel, pour que ce dernier décide d'effectuer ou non le lancement du programme de traitement. L'avantage de ce dispositif est qu'il prévient non seulement la mise en court-circuit des électrodes, mais également l'utilisation d'une simple charge fictive à résistance ohmique. Il n'accepte en effet qu'une charge combinée ohmique-capacitive proche d'une charge corporelle réelle.

## Revendications

1. Dispositif de stimulation électrique neuro-musculaire comprenant au moins un élément de stimulation (2; 50) portable et autonome commandé par un support d'information (4; 53) amovible et interchangeable, préalablement programmé en fonction du traitement de chaque patient considéré, équipé d'au moins une sortie comportant au moins une paire d'électrodes (6, 6'; 56, 56') agencées pour être appliquées sur la partie à traiter du corps du patient et à transmettre des trains d'impulsions électriques de stimulation destinées à provoquer des contractions et un exercice ou un entraînement musculaire, caractérisé en ce que l'élément de stimulation comporte une circuiterie électronique, hardware, de façon continue et progressive, pendant la séance de traitement, qui transmet des trains d'impulsions électriques de stimulation au moyen des dites électrodes et des moyens de commande logiciels mémorisés sur le support d'information amovible qui commandent et supervisent la transmission desdits trains d'impulsions électriques de stimulation et comportant des moyens programmables pour faire évoluer entièrement sous la contrôle du logiciel, la valeur de chaque paramètre du traitement de stimulation entre des valeurs de référence préalablement programmées et pour permettre un couplage interactif de tout paramètre de traitement avec un autre paramètre de traitement.

2. Dispositif selon la revendication 1, caractérisé en ce que l'élément de stimulation comporte un microprocesseur générateur d'impulsions électriques, à programmation dynamique déroulante en fonction du temps de déroulement du programme de traitement.

3. Dispositif selon la revendication 2, caractérisé en ce que le microprocesseur asservit une cellule d'isolation (21) comprenant un amplificateur conformateur de sortie du courant de stimulation vers les électrodes, de façon à constituer un étage redresseur, la cellule d'isolation étant complétée par un étage commutateur-inverseur électronique (26) de la polarité, commandé par le logiciel, par l'intermédiaire de la cellule d'isolation (22).

4. Dispositif selon l'une des Revendications 2 ou 3, caractérisé en ce que les impulsions électriques générées par le microprocesseur sont progressivement adaptables pour provoquer des séries consécutives de contractions musculaires permettant la mise en jeu à choix d'un métabolisme énergétique aérobie, anaérobie alactique ou anaérobie lactique.

5. Dispositif selon l'une des revendications 2 à 4, caractérisé en ce que l'élément de stimulation comporte des moyens asservis au logiciel et au microprocesseur pour coupler les différents paramètres de stimulation et les rendre intercatifs en fonction du déroulement automatique du programme de traitement, et en particulier pour asservir l'intensité du courant de stimulation à la fréquence des impulsions électriques délivrées, de façon à assurer un travail musculaire physiologique et sans aucun risque pour l'appareil tendino-musculaire.

6. Dispositif selon l'une des revendications 2 à 5, caractérisé en ce que l'élément de stimulation comporte des moyens asservis au logiciel et au microprocesseur pour mémoriser l'intensité du courant électrique de stimulation assignée par le thérapeute ou par le patient et délivré aux électrodes en fonction du déroulement automatique du programme de traitement, et pour restituer ensuite progressivement la même intensité à l'occasion d'un traitement ultérieur.

7. Dispositif selon l'une des revendications 2 à 6, caractérisé en ce que l'élément de stimulation comprend des moyens asservis au logiciel et au microprocesseur agencés pour le contrôle de l'observance du traitement par le patient, lesdits moyens mesurant de façon constante l'impédance de la charge entre les deux électrodes de chaque paire d'électrodes pendant toute la durée du traitement et effectuant une mesure-test préalable en générant une impulsion de courant constant d'intensité subliminaire et d'une durée suffisante pour permettre la comparaison de la pente de la tension de ce signal avec une valeur préprogrammée.

8. Dispositif selon l'une des revendications précédentes, caractérisé en ce que les différents canaux de sortie sont programmables indépendemment les uns des autres et sont agencés de façon à permettre une interdépendance de leur activité, tout paramètre d'un canal de sortie pouvant asservir un autre canal de sortie, de manière à permettre la programmation d'activités musculaires multiples.

9. Dispositif selon l'une des revendications précédentes, caractérisé en ce que l'élément de stimulation comporte des moyens de sécurité, actionnables par le patient, pour régler exclusivement l'intensité de la stimulation dans les limites programmées.

10. Dispositif selon l'une des revendications précédentes, caractérisé en ce que l'élément de stimulation comprend un écran d'affichage LCD du type à matrice de points, fonctionnant en mode TV et géré par le logiciel d'exploitation et par le logiciel constituant le programme de traitement, de façon à permettre le contrôle de toutes les fonctions du dispositif et de la stimulation.

11. Dispositif selon l'une des revendications précédentes, caractérisé en ce que l'élément de stimulation comporte des touches de fonction (56) asservies au logiciel et permettant de personnaliser le programme.

12. Dispositif selon l'une des revendications 10 ou 11, caractérisé en ce que l'écran (55) et/ou les touches de fonction (56) sont disposés sur un élément rabattable (54) fonctionnant comme un couvercle de protection.

13. Dispositif selon l'une des revendications précédentes, caractérisé en ce que le stimulateur comporte un logement (2) muni d'un connecteur pour l'insertion de cartes-programmes.

14. Dispositif selon la revendication 13, caractérisé en ce qu'il comporte des cartes-programmes préétablies (70), serveuses de programmes standards de traitements, chaque programme étant spécifique à une indication médicale précise, le stimulateur étant agencé de façon à permettre à l'utilisateur de choisir un des programmes et de mémoriser ce programme sur une carte-programme-patient (53) qu'il introduit dans le logement en remplacement de la carte-programmes préétablie (70).

15. Dispositif selon l'une des revendications précédentes, caractérisé en ce que l'élément de stimulation comporte quatre sorties électriquement indépendantes et pouvant être couplées de manière interactive par les couplages prévus par le logiciel entre les différents paramètres de stimulation en fonction du temps de déroulement du programme de traitement.

16. Dispositif selon l'une des revendications précédentes, caractérisé en ce que l'élément de stimulation est équipé d'un connecteur à câble amovible (3) permettant une liaison sérielle du type RS 232C, de façon à permettre sa connection à un ordinateur (1).

17. Dispositif selon l'une des revendications précédentes, caractérisé en ce que l'élément de stimulation comporte un connecteur multifonctions (61) agencé pour permettre la connexion du stimulateur avec un chargeur de batteries ou une batterie d'alimentation d'appoint, ou avec différents éléments périphériques de commande.

18. Dispositif selon l'une des revendications précédentes, caractérisé en ce qu'il est agencé de façon à permettre une liaison du type bus de données pour la commande externe interactive des canaux de sortie d'un ou de plusieurs stimulateurs couplés en réseau, éventuellement commandes par une unité de commande externe.

## Claims

1. An electrical neuromuscular stimulation device, comprising at least one portable autonomous stimulation unit (2; 50), controlled by a detachable and interchangeable information medium (4; 53), previously programmed as a function of the treatment for each respective patient, equipped with at least one output with at least one pair of electrodes (6, 6'; 56, 56') designed to be applied to the part of the patient's body to be treated and to transmit trains of electrical stimulation impulses intended to cause contractions and muscular exercise or training, characterised in that the stimulation unit comprises a hardware electronic circuitry, entirely under the supervision of the software, transmitting trains of electrical stimulation impulses through said electrodes and software control means memorised on the detachable information medium, said software controlling and supervising the transmission of said trains of electrical stimulation impulses and comprising programmable means for enabling the value of each parameter of the stimulation treatment to evolve continuously and progressively in the course of the treatment session between previously programmed reference values and for enabling interactive coupling between any parameter of treatment and an other parameter of treatment.

2. A device according to claim 1, characterised in that the stimulation unit comprises an electrical impulse generating microprocessor, enabling developing dynamic programming as a function of the performance time of the program of treatment.

3. A device according to claim 2, characterised in that the microprocessor controls an insulation cell (21) comprising an output pulse shaper-amplifier of the stimulation current towards the electrodes, so as to form a rectifier stage, with the insulation cell being supplemented by an electronic polarity change-over switch stage (26) controlled by the software, by means of the insulation cell (22).

4. A device according to one of claims 2 or 3, characterised in that the electrical impulses generated by the microprocessor can be progressively adapted to cause consecutive series of muscular contractions enabling the selection of an energetic aerobic, anaerobic alactic or anaerobic lactic metabolism.

5. A device according to one of claims 2 to 4, characterised in that the stimulation unit comprises means subordinate to the software and to the microprocessor for coupling the different stimulation parameters and to make them interactive as a function of the automatic progress of the program of treatment, and in particular to make the intensity of the stimulation current subordinate to the frequency of the electrical impulses supplied, so as to ensure physiological muscular work and without any risk for the tendino-muscular appliance.

6. A device according to one of claims 2 to 5, characterised in that the stimulation unit comprises means subordinate to the software and to the microprocessor for memorising the intensity of the electrical stimulation current assigned by the therapist or by the patient and supplied to the electrodes as a function of the automatic progress of the program of treatment, and to then progressively restore the same intensity at the time of a subsequent treatment.

7. A device according to one of claims 2 to 6, characterised in that the stimulation unit comprises means subordinate to the software and to the microprocessor designed to monitor the observance of the treatment by the patient, the said means measuring constantly the impedance of the charge between the two electrodes of each pair of electrodes throughout the entire treatment and carrying out a preliminary test measurement by generating a constant current impulse of a subliminal intensity and of an adequate duration to allow the comparison of the voltage slope of this signal with a preprogrammed value.

8. A device according to one of the above claims, characterised in that the different output channels can be programmed independently from one another and are designed so as to allow an interdependence of their activity, with every parameter of an output channel being able to control another output channel, so as to allow the programming of multiple muscular activities.

9. A device according to one of the above claims, characterised in that the stimulation unit comprises safety means, which can be operated by the patient, to adjust exclusively the intensity of the stimulation within programmed limits.

10. A device according to one of the above claims characterised in that the stimulation unit comprises a matrix dot LCD screen, operating in TV mode and controlled by the operating software and by the software constituting the program of treatment, so as to enable the monitoring of all the functions of the device and of the stimulation.

11. A device according to one of the above claims, characterised in that the simulation unit comprises function keys (56) subordinate to the software and enabling the program to be personalised.

12. A device according to one of claims 10 to 11, characterised in that the screen (55) and/or the function keys (56) are disposed on a unit (54) which can be folded down and which serves as a protective cover.

13. A device according to one of the above claims, characterised in that the stimulator comprises a slot (2) equipped with a connector for the insertion of program-cards.

14. A device according to claim 13, characterised in that it comprises previously established program-cards (70), servers of standard programs of treatment, with each program being specific to a precise medical indication, the stimulator being designed so as to allow the user to choose one of the programs and to memorise this program on a patient program-card (53) which he introduces into the slot by replacing the previously established program-card (70).

15. A device according to one of the above claims, characterised in that the stimulation unit comprises four outputs which are electrically independent and which can be coupled interactively by the connections specified by the software between the different stimulation parameters as a function of the performance time of the program of treatment.

16. A device according to one of the above claims, characterised in that the stimulation unit is equipped with a connector having a detachable cable (3) which permits an RS 232C type serial link, so as to enable its connection to a computer (1).

17. A device according to one of the above claims, characterised in that the stimulation unit comprises a multifunction connector (61) designed to enable the connection of the stimulator to a battery charger or to an auxiliary power supply battery, or to different peripheral control units.

18. A device according to one of the above claims, characterised in that it is designed so as to enable a data bus type connection for the external interactive control of the output channels of one or several stimulators connected in network, possibly controlled by an external control unit.

## Patentansprüche

1. Vorrichtung zur elektrischen Stimulation von Muskelnerven mit zumindest einer tragbaren autonomen Stimulationseinrichtung (2; 50), die durch einen entfernbaren und austauschbaren Informationsträger (4; 53) gesteuert ist, welcher im vorhinein in Abhängigkeit der Behandlung eines jeweiligen Patienten programmierbar ist, wobei die Einrichtung mit mindestens einem Ausgang ausgerüstet ist, an den zumindest ein Elektrodenpaar (6, 6'; 56, 56') angeschlossen ist, die an dem zu behandelnden Bereich des Patientenkörpers anlegbar sind und elektrische Stimulationsimpulszüge übertragen können, durch die Kontraktionen sowie Übung oder Training der Muskeln veranlasst werden können, dadurch gekennzeichnet, dass die Stimulationseinrichtung eine in der Gesamtheit unter Kontrolle der Software stehende elektronische Hardware-Schaltung aufweist, die elektrische Stimulationsimpulszüge mit Hilfe der Elektroden überträgt und Steuereinrichtungen für Software, die auf dem austauschbaren Informationsträger gespeichert ist, aufweist, die die Übertragung der elektrischen Stimulationsimpulszüge steuern und überwachen, und dass programmierbare Einrichtungen vorgesehen sind, um während der Behandlungsdauer kontinuierlich und progressiv den Wert jedes Parameters der Stimulationsbehandlung zwischen vorab programmierbaren Referenzwerten langsam zu verändern und um eine interaktive Verknüpfung des jeweiligen Behandlungsparameters mit einem anderen Behandlungsparameter zu erlauben.

2. Vorrichtung nach Anspruch 1, dadurch gekennzeichnet, dass die Stimulationseinrichtung einen Mikroprozessor-Generator für elektrische Impulse mit einer dynamischen Programmierung aufweist, die als Funktion des Zeitablaufes des Behandlungsprogrammes abläuft.

3. Vorrichtung nach Anspruch 2, dadurch gekennzeichnet, dass der Mikroprozessor eine Isolationszelle (21) steuert, die einen Formverstärker für den Ausgang des Stimulationsstromes zu den Elektroden aufweist, um eine Gleichrichterstufe zu bilden, wobei die Isolationszelle durch eine elektronische Schaltstufe (26) zum Wechseln der Polarität ergänzt ist, die durch die Software unter Zwischenschaltung der Isolationszelle (22) gesteuert ist.

4. Vorrichtung nach einem der Ansprüche 2 oder 3, dadurch gekennzeichnet, dass die elektrischen, durch den Mikroprozessor erzeugten Impulse progressiv anpassbar sind, um aufeinanderfolgende Serien von Muskelkontraktionen hervorzurufen, die wahlweise einen energetisch aeroben, anaeroben antilaktischen oder anaeroben laktischen Metabolismus ermöglichen.

5. Vorrichtung nach einem der Ansprüche 2 bis 4, dadurch gekennzeichnet, dass die Stimulationseinrichtung von der Software und dem Mikroprozessor gesteuerte Einrichtungen aufweisen, um unterschiedliche Stimulationsparameter zu verknüpfen und sie interaktiv miteinander in Funktion des automatischen Behandlungsprogrammablaufes zu verbinden und insbesondere um die Intensität des Stimulationsstromes in Abhängigkeit der Frequenz der gelieferten elektrischen Impulse zu steuern, um auf diese Weise eine physiologische Muskelarbeit ohne Risiko für den Sehnen-Muskel-Apparat sicherzustellen.

6. Vorrichtung nach einem der Ansprüche 2 bis 5, dadurch gekennzeichnet, dass die Stimulationseinrichtung von der Software und dem Mikroprozessor gesteuerte Einrichtungen aufweisen, um die Intensität des elektrischen, von dem Therapeuten oder dem Patienten eingestellten und an die Elektroden abgegebenen Stimulationsstromes in Funktion des automatischen Behandlungsprogrammablaufes zu speichern und um anschließend progressiv dieselbe Intensität bei einer folgenden Behandlungen abzugeben.

7. Vorrichtung nach einem der Ansprüche 2 bis 6, dadurch gekennzeichnet, dass die Stimulationseinrichtung von der Software und dem Mikroprozessor gesteuerte Einrichtungen aufweisen, die für die Kontrolle der Einhaltung der Behandlung durch den Patienten ausgelegt sind, wobei diese Einrichtungen ständig die Impedanz der zwischen den beiden Elektroden eines jeden Elektrodenpaares während der gesamten Dauer der Behandlung messen und einen voreinstellbaren Meßtest bewirken, indem ein Impuls eines Konstantstromes mit niedriger Intensität und mit einer Zeitdauer erzeugt wird, die ausreicht, um den Vergleich des Anstieges der Spannung dieses Signales mit einem vorprogrammierten Wert zu erlauben.

8. Vorrichtung nach einem der vorhergehenden Ansprüche, dadurch gekennzeichnet, dass die verschiedenen Ausgangskanäle unabhängig voneinander programmierbar und derart ausgelegt sind, dass eine Wechselbeziehung ihrer Aktivität erlaubt ist, wobei jeder Parameter eines Ausgangskanales einen anderen Ausgangskanal steuern kann, um die Programmierung vielfältiger Muskelaktivitäten zu erlauben.

9. Vorrichtung nach einem der vorhergehenden Ansprüche, dadurch gekennzeichnet, dass die Stimulationseinrichtung Sicherheitseinrichtungen aufweist, die durch den Patienten aktivierbar sind und mit denen ausschließlich die Stimulationsintensität in vorgegebenen Grenzen einstellbar ist.

10. Vorrichtung nach einem der vorhergehenden Ansprüche, dadurch gekennzeichnet, dass die Stimulationseinrichtung einen LCD-Anzeigeschirm mit einer Matrix-Punktdarstellung aufweist, der nach TV-Art arbeitet und durch die Auswertesoftware und die das Behandlungsprogramm zur Verfügung stellende Software angesteuert wird, um die Überwachung aller Funktionen der Vorrichtung und der Stimulation zu erlauben.

11. Vorrichtung nach einem der vorhergehenden Ansprüche, dadurch gekennzeichnet, dass die Stimulationseinrichtung von der Software gesteuerte Funktionstasten (56) aufweist, die eine Personalisierung des Programmes erlauben.

12. Vorrichtung nach einem der Ansprüche 10 oder 11, dadurch gekennzeichnet, dass der Schirm (55) und/oder die Funktionstasten (56) auf einem Klappteil (54) angeordnetsind, das als Sicherheitsdeckel dient.

13. Vorrichtung nach einem der vorhergehenden Ansprüche, dadurch gekennzeichnet, dass die Stimulationseinrichtung eine mit einem Stecker versehene Aufnahme (2) zum Einfügen von Programmkarten aufweist.

14. Vorrichtung nach Anspruch 13, dadurch gekennzeichnet, dass vorgefertigte Programmkarten (70) vorgesehen sind, die Standard-Behandlungsprogramme liefern, wobei jedes Programm für eine präzise medizinische Indikation spezifiziert ist, wobei die Stimulationseinrichtung so eingerichtet ist, um wahlweise die Verwendung eines der Programme zu erlauben und dieses Programm auf einer Patienten-Programm-Karte (53) zu speichern, die in die Aufnahme anstatt der vorgefertigten Programmkarte (70) eingeführt wird.

15. Vorrichtung nach einem der vorhergehenden Ansprüche, dadurch gekennzeichnet, dass die Stimulationseinrichtung vier elektrisch voneinander unabhängige Ausgänge aufweist, die durch die Software durch interaktive Verknüpfungen zwischen unterschiedlichen Stimulationsparametern in Abhängigkeit des Zeitablaufes des Behandlungsprogrammes verknüpft werden können.

16. Vorrichtung nach einem der vorhergehenden Ansprüche, dadurch gekennzeichnet, dass die Stimulationseinrichtung mit einem austauschbaren Verbindungskabel (3) ausgerüstet ist, das den Anschluß einer seriellen Schnittstelle vom Typ RS 232C erlaubt, um die Verbindung mit einem Rechner (1) herstellen zu können.

17. Vorrichtung nach einem der vorhergehenden Ansprüche, dadurch gekennzeichnet, dass die Stimulationseinrichtung einen Multifunktionsstecker (61) aufweist, mit dem die Verbindung der Stimulationseinrichtung mit einem Batterieladegerät oder einer Versorgungsbatterie oder mit verschiedenen Peripherie-Steuergeräten möglich ist.

18. Vorrichtung nach einem der vorhergehenden Ansprüche, dadurch gekennzeichnet, dass sie ausgebildet ist, um eine Verbindung mit einem Datenbus für die externe interaktive Steuerung zwischen den Ausgangskanälen eines oder mehrerer in einem Netz miteinander verbundenen Stimulationseinrichtungen zu erlauben, die gegebenenfalls durch eine externe Steuereinrichtung gesteuert werden.
